# EUROPÄISCHE PATENTANMELDUNG

(11) **EP 1 270 535 A2**
(43) Veröffentlichungstag der Anmeldung: **02.01.2003**
(21) Anmeldenummer: 02012763.5
(22) Anmeldetag: 08.06.2002
(51) Int. Cl.: C07B 37/04, C07C 45/44, C07C 319/20, C07D 213/50, C07D 307/12

(54) **Verfahren zur Herstellung von substituierten aromatischen Verbindungen**

(30) Priorität: 20.06.2001 DE 10129765; 09.11.2001 DE 10155209
(71) Anmelder: Clariant GmbH, 65929 Frankfurt am Main (DE)
(72) Erfinder: Meudt, Andreas, Dr., 65439 Fl-rsheim-Weilbach (DE); Erbes, Michael, 65931 Frankfurt (DE); Forstinger, Klaus, Dr., 64832 Babenhausen (DE)
(74) Vertreter: Hütter, Klaus, Dr.

(57) **Zusammenfassung**

Verfahren zur Herstellung von Verbindungen der Formel (II), 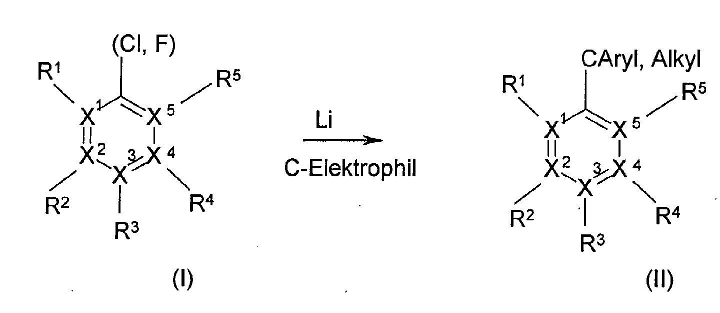 worin die Substituenten R¹ bis R⁵ unabhängig voneinander für H, CH₃, geradkettiges oder verzweigtes C₁-C₈-Alkyl, insbesondere C₁-C₄-Alkyl, CH(OC₁-C₅-Alkyl)₂, CH(C₁-C₅-Alkyl)(OC₁-C₅-Alkyl), CH₂(OC₁-C₅-Alkyl), CH(CH₃)(OC₁-C₅-Alkyl), C₁-C₈-Alkoxy, insbesondere C₁-C₄-Alkoxy, N(C₁-C₅-Alkyl)₂, Phenyl, substituiertes Phenyl, Aryl, Heteroaryl, S(C₁-C₅-Alkyl) oder für einen Rest C_{Aryl,Alkyl} stehen, und die Symbole X^{1 bis 5} für Kohlenstoff oder maximal zwei benachbarte X¹⁻⁵ für Stickstoff oder X¹R¹ und X²R² zusammen für O, NH, N(C₁-C₅-Alkyl), N(C=O-C₁-C₅-Alkyl), N(SiR₃)₂ oder S stehen,
oder wobei benachbarte Reste R¹ bis R⁵ folgende Struktureinheit bilden, 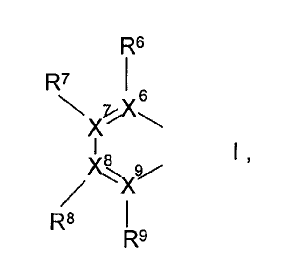 wobei X⁶ bis X⁹ und R⁶ bis R⁹ die gleiche Bedeutung haben wie X¹ bis X⁵ und R¹ bis R⁵
und
der Rest C_{Aryl}, _{Alkyl} für geradkettiges oder verzweigtes, substituiertes oder unsubstituiertes C₁-C₈-Alkyl, 1-Hydroxyalkyl mit 1 bis 8 C-Atomen, CN, 2-Hydroxyalkyl mit 2 bis 5 C-Atomen, 3-Hydroxyalkyl mit 3 bis 5 C-Atomen, 1-NHR-Alkyl mit 1 bis 5 C-Atomen, CH(OC₁-C₅-Alkyl)₂, C(C₁-C₅-Alkyl)(OC₁-C₅-Alkyl), CH₂(OC₁-C₅-Alkyl), CH(CH₃)(OC₁-C₅-Alkyl), C₁-C₅-Alkoxy, N(C₁-C₅-Alkyl)₂, Phenyl, substituiertes Phenyl, Aryl, Heteroaryl, CO₂H, CO₂Alkyl, (C=O)_{0.5}, substituierte 1-Vinylalkyle, CH₃-C(=O), R-C(=O) oder CHO steht,
durch Umsetzung von Chlor- oder Fluoraromaten der Formel (I) mit Kohlenstoff-Elektrophilen (C-Elektrophilen) und Lithiummetall.

## Beschreibung

Die Erfindung betrifft ein Verfahren zur Knüpfung von Kohlenstoff-Kohlenstoff-Bindungen ausgehend von Chlor- oder Fluoraromaten durch Umsetzung mit Lithiummetall und einem Kohlenstoff-Elektrophil, wodurch eine breite Palette von Alkyl- oder Aryl-substituierten Aromaten und Heteroaromaten erhalten werden können.

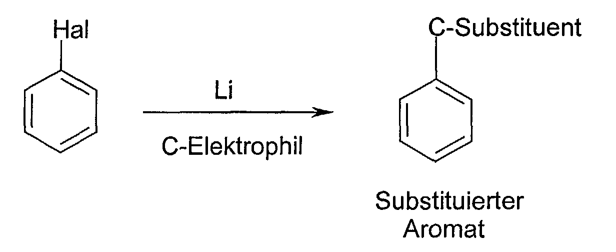

Aus derartigen Umwandlungen von Chlor- und Fluoraromaten in Alkyl- und Aryl-substituierte Aromaten und Heteroaromaten lassen sich beispielsweise eine sehr breite Palette vielseitig anwendbarer Zwischenprodukte und Wirkstoffe der agrochemischen und pharmazeutischen Industrie herstellen, an denen ein immenses wirtschaftliches Interesse besteht.

Die Umwandlung von Halogenaromaten in Alkyl- oder Aryl-substituierte Aromaten ist in zahllosen Publikationen auf vielen verschiedenen Wegen beschrieben, für sehr viele dieser Reaktionen existieren sogar allgemeine Arbeitsvorschriften, nach denen sich die jeweiligen Zielverbindungen in guten Ausbeuten erhalten lassen.

Die wichtigste und sehr breit anwendbare allgemeine Vorgehensweise ist die Umsetzung der Halogenaromaten zu Grignard-Verbindungen, die sich dann nachfolgend mit einer breiten Palette an C-Elektrophilen zu den Zielverbindungen umsetzen lassen. Die Reaktion von Brom- oder lodaromaten zu Grignard-Verbindungen gelingt bei Abwesenheit von mit der Grignard-Funktionalität reagierenden Resten im Halogenaromaten in nahezu allen Fällen in guten bis sehr guten Ausbeuten. Zu beachten ist aber, dass Brom- und lodaromaten fast immer bedeutend teurer als die entsprechenden Chloraromaten sind, so dass es für konkurrenzfähige technische Herstellverfahren ein Muss ist, letztere einzusetzen. Leider gibt es zahlreiche Fälle, in denen ausgehend von entsprechenden Chlor- oder Fluoraromaten die Grignard-Verbindungen nur in schlechten Ausbeuten, in speziellen, oftmals teuren Lösungsmitteln oder unter Anwendung teurer Aktivierungsmethoden für das Magnesium-Metall herstellbar sind. Dies gilt beispielsweise für 1-Chlornaphthalin, bei dem als Beispiel für eine spezielle und teure Arbeitstechnik der Einsatz von Rieke-Magnesium erforderlich ist, um überhaupt nennenswerte Umsätze erreichen zu können.

Für diese Fälle gibt es kaum eine Alternative zur Verwendung der Brom- oder lodaromaten, denn auch die Metallierung mit z.B. Butyllithium gelingt mit Chloraromaten nicht, und auch die umgekehrte Vorgehensweise, z. B. eine Kupplung des Halogenaromaten mit einem nucleophilen Reagens wie z. B. einem Alkyl- oder Arylgrignard oder einer Boronsäure gelingt meist nur mit den aktiveren Brom- oder lodaromaten. In den wenigen beschriebenen Kupplungsreaktionen von Metallaten mit Chlor- oder Fluorbenzolen ist es erforderlich, oft große Mengen speziell entwickelter und meist sehr teurer Liganden einzusetzen, so dass dies meist keine ernsthaft in Erwägung zu ziehende Alternative ist.

Ein weiterer bedeutender Nachteil des erwähnten Prozesses ist apparativer Natur. Aus verfahrenstechnischer Sicht ist die Herstellung von Grignard-Verbindungen aus Chlor- oder Fluoraromaten auch deshalb problematisch, weil die Reaktion häufig zunächst gar nicht und dann sehr plötzlich und oft unkontrolliert anspringt. Es wird oft beobachtet, dass die Zeit bis zum Anspringen sehr stark von der Qualität der eingesetzten Lösungsmittel abhängt (beispielsweise Wassergehalt, Gehalt an Radikalbildnern und Metallionen etc.). Für einen kontrollierten technischen Prozess sind dies keine optimalen Voraussetzungen.

Das größte Problem und der größte Kostentreiber bei der Herstellung von Alkyl- und Aryl-substituierten Aromaten aus Chlor- und Fluorbenzolen ist aber der erhebliche apparative Aufwand. Da die daraus erhältlichen Arylmetallate wie beispielsweise die schon mehrfach erwähnten Arylgrignard-Verbindungen nur in ganz wenigen Fällen und dann zu horrenden Preisen kommerziell erhältlich sind, muss man in einem ersten, meist auf Rückflusstemperatur gehaltenen Kessel die Grignard-Verbindung herstellen, nach Erreichen vollständigen Umsatzes darin abkühlen, in einem zweiten Kessel das entsprechende C-Elektrophil vorlegen, dieses aufgrund der hohen Reaktivität der Arylmetallate meist auf möglichst niedrige Temperaturen abkühlen, anschließend die ebenfalls gekühlte Grignard-Verbindung zudosieren, auftauen, in einem dritten Kessel hydrolysieren (Kessel 1 und 2 müssen absolut wasserfrei bleiben) und die Aufarbeitung in diesem dritten oder einem weiteren Kessel durchführen. Durch die gleichzeitige Belegung mehrerer Kessel und die notwendigen, bei größeren Mengen langwierigen Aufheiz- und Abkühlphasen erreicht man nur mäßige Raum/Zeit-Ausbeuten und insgesamt hohe Herstellkosten.

Die Herstellung anderer metallorganischer Reagenzien, z. B. basierend auf den Metallen Zink, Aluminium, Natrium, Kalium oder Silizium stellt ebenfalls keine sinnvolle Alternative dar, da die Metalle für gewöhnlich zu unreaktiv sind, um Chloraromaten einsetzen zu können (Zn, Al, Si), oder da die entstehenden Metallate sehr leicht zu Biarylen und sonstigen Folgeprodukten kuppeln bzw. zu intramolekularen Umlagerungen neigen (K, Na, schon Tolylnatrium bzw. -kalium lagern zu Benzylmetallaten um).

Es wäre daher sehr wünschenswert, ein Verfahren zu haben - unter Beibehaltung der Rohstoffe Chlor- oder Fluoraromat und C-Elektrophil - bei dem idealerweise alle Verfahrensschritte bei ein- und derselben Temperatur oder einer nur geringfügig verschiedenen Temperatur durchgeführt werden und dadurch lange Temperierungsphasen vermieden werden können. Noch wichtiger wäre aber die Möglichkeit, die Herstellung des metallorganischen Reagenzes im gleichen Kessel wie die Umsetzung mit dem C-Elektrophil durchführen zu können. Da aber die Herstellung der Grignard-Verbindung meist bei Rückflusstemperatur des verwendeten Lösungsmittels, die Addition des Reaktionspartner aber aus Selektivitätsgründen bei Temperaturen < 0 °C durchgeführt werden muss, erscheint dies über eine Grignard-Route nicht möglich.

Ein weiterer, häufig beschrittener Weg zur Herstellung von Alkyl- oder Aryl-substituierten Aromaten ist die Umsetzung von lithiierten Aromaten und Heteroaromaten mit C-Elektrophilen. Die Herstellung von Lithioaromaten kann ebenfalls auf zahlreichen Wegen erfolgen. Beispielsweise ist die Umsetzung von Brom- und lodaromaten mit Butyllithium ein Standardweg, um Lithioaromaten zu generieren. Dieser Austausch lässt sich bei tiefen Temperaturen durchführen, bei denen dann auch die Umsetzungen mit C-Verbindungen hochselektiv durchgeführt werden können.

Leider lässt sich diese Umsetzung aber nicht mit Chloraromaten durchführen, da diese mit sehr wenigen Ausnahmen nicht mit Butyllithium reagieren. Aufgrund dieser Tatsache und des hohen Preises für Butyllithium resultiert trotz der genannten Vorteile in der Gesamtbilanz kein besonders ökonomisches Verfahren.

Aus dem Stand der Technik sind verschiedene Methoden zur Herstellung von Lithiumverbindungen bekannt. Es wurde aber bisher noch kein Gesamtprozess zum Austausch von Chlor bzw. Fluor gegen Alkyl- oder Arylreste, der allen oben beschriebenen Anforderungen genügt, beschrieben.

Es bestand daher das Bedürfnis, ein Verfahren zur Herstellung von Verbindungen der Formel (I) zu entwickeln, das von kommerziell leicht erhältlichen und günstigen Chlor- oder Fluorverbindungen ausgeht, das die benötigten Aryl- oder Alkylsubstituierten Aromaten und Heteroaromaten in guten Ausbeuten und hohen Reinheiten zugänglich macht und das dabei gleichzeitig verfahrenstechnisch einfach, effizient und kostengünstig arbeitet. Letzteres beinhaltet gleichzeitig die Durchführung aller Verfahrensschritte ausschließlich Hydrolyse und Aufarbeitung bei ein- und derselben Temperatur und nach Möglichkeit in ein- und demselben Reaktionskessel. Idealerweise ermöglicht das Verfahren daneben auch die direkte Herstellung der Zielverbindungen durch einfaches Rühren von Chloraromat, Metall und C-Elektrophil in einem geeigneten Lösungsmittel.

Die vorliegende Erfindung löst alle diese Aufgaben und betrifft ein Verfahren zur Herstellung von Verbindungen der Formel (II), 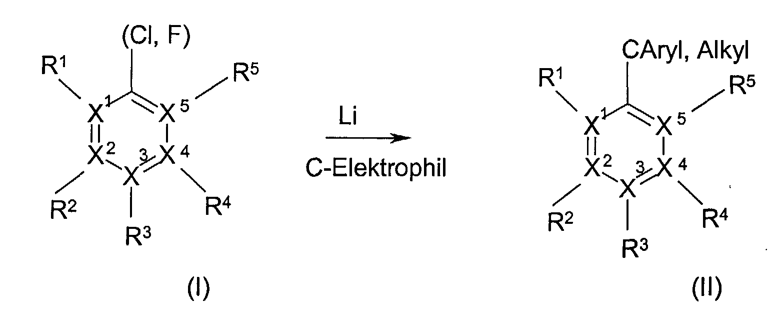 worin die Substituenten R¹ bis R⁵ unabhängig voneinander für H, CH₃, geradkettiges oder verzweigtes C₁-C₈-Alkyl, insbesondere C₁-C₄-Alkyl, CH(OC₁-C₅-Alkyl)₂, CH(C₁-C₅-Alkyl)(OC₁-C₅-Alkyl), CH₂(OC₁-C₅-Alkyl), CH(CH₃)(OC₁-C₅-Alkyl), C₁-C₈-Alkoxy, insbesondere C₁-C₄-Alkoxy, N(C₁-C₅-Alkyl)₂, Phenyl, substituiertes Phenyl, Aryl, Heteroaryl, Pyridyl, substituiertes Pyridyl, Chinolinyl, substituiertes Chinolinyl, Naphthyl, substituiertes Naphthyl, Furanyl, substituiertes Furanyl, Benzofuranyl, substituiertes Benzofuranyl, S(C₁-C₅-Alkyl) oder für einen Rest C_{Aryl, Alkyl} stehen und
die Symbole X^{1 bis 5} für Kohlenstoff oder maximal zwei benachbarte X¹⁻⁵ für Stickstoff (N-Sechsring-Heterocyclen) oder X¹R¹ und X²R² zusammen für O, NH, N(C1-C5-Alkyl), N(C=O-C1-C5-Alkyl), N(SiR3)₂ oder S (Fünfring-Heterocyclen) stehen,
oder wobei benachbarte Reste R¹ bis R⁵ folgende Struktureinheit bilden, 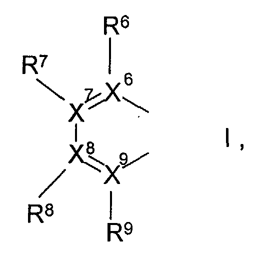 wobei X⁶ bis X⁹ und R⁶ bis R⁹ die gleiche Bedeutung haben wie X¹ bis X⁵ und R¹ bis R⁵ und
der Rest C_{Aryl, Alkyl} für CH₃, geradkettiges oder verzweigtes, substituiertes oder unsubstituiertes C₁-C₈-Alkyl, insbesondere C₁-C₄-Alkyl, 1-Hydroxyalkyl mit 1 bis 8 C-Atomen, CN, 2-Hydroxyalkyl mit 2 bis 5 C-Atomen, 3-Hydroxyalkyl mit 3 bis 5 C-Atomen, 1-NHR-Alkyl mit 1 bis 5 C-Atomen, CH(OC₁-C₅-Alkyl)₂, C(C₁-C₅-Alkyl)(OC₁-C₅-Alkyl), CH₂(OC₁-C₅-Alkyl), CH(CH₃)(OC₁-C₅-Alkyl), C₁-C₅-Alkoxy, insbesondere C₁-C₄-Alkoxy, N(C₁-C₅-Alkyl)₂, Phenyl, substituiertes Phenyl, Aryl, Heteroaryl, CO₂H, CO₂Alkyl, (C=O)_{0.5} (was der Struktureinheit Ar-CO-CO-Ar entsprechen würde), substituierte 1-Vinylalkyle, CH₃-C(=O), R-C(=O) oder CHO steht, durch Umsetzung von Chlor- oder Fluoraromaten der Formel (I) mit Kohlenstoff-Elektrophilen (C-Elektrophil) und Lithiummetall.

Das Kohlenstoff-Elektrophil stammt insbesondere aus einer der folgenden Kategorien:
Aryl- oder Alkylcyanate (C_{Aryl,Alkyl} = CN)
Oxiran, substituierte Oxirane (C_{Aryl,Alkyl} = CH₂CH₂OH, CR₂CR₂OH) mit R = R¹ (gleich oder verschieden)
Azomethine (C_{Aryl,Alkyl} = CR¹₂-NR'H)
Nitroenolate (C_{Aryl,Alkyl} = Oxime)
Immoniumsalze (C_{Aryl,Alkyl} = Amine)
Halogenaromat, Aryltriflate, andere Arylsulfonate (C_{Aryl,Alkyl} = Aryl, Heteroaryl) Kohlendioxid (C_{Aryl,Alkyl} = COOH)
Kohlenmonoxid (C_{Aryl,Alkyl} = (-CO-)_{0,5})
Aldehyde, Ketone (C_{Aryl,Alkyl} = CHR¹-OH, CR¹₂-OH;
α,β-ungesättigte Aldehyde/Ketone (C_{Aryl,Alkyl} = CH(OH)-Vinyl, CR¹(OH)-Vinyl)
Ketene (C_{Aryl,Alkyl} = C(=O)CH₃ bei Keten, C(=O)-R¹ bei substituierten Ketenen) Alkali- und Erdalkalisalze von Carbonsäuren (C_{Aryl,Alkyl} = CHO bei Formiaten, COCH₃ bei Acetaten, R¹CO bei R¹COOMet)
Aliphatische Nitrile (C_{Aryl,Alkyl} = COCH₃ bei Acetonitril, R¹CO bei R¹CN)
Aromatische Nitrile (C_{Aryl,Alkyl} = COAr')
Amide (C_{Aryl,Alkyl} = CHO bei HCONR₂, C(=O)R bei RCONR'₂)
Ester (C_{Aryl,Alkyl} = [C(OH)R¹]_{0,5}) oder
Alkylierungsmittel (C_{Aryl,Alkyl} = Alkyl).

Das erfindungsgemäße Verfahren eröffnet eine Methode, um die kostengünstigen und einfach zugänglichen Chlor- und Fluoraromaten als ideale Startmoleküle in eine breite Palette von Verbindungen unter hoher Wertschöpfung umzuwandeln.

Eine bevorzugte Ausführungsform ist das gleichzeitige Rühren von C-Elektrophil, Chlor- oder Fluoraromat und Lithiummetall in einem geeigneten Lösungsmittel (Eintopfvariante), wobei nach entsprechender Aufarbeitung (meist Hydrolyse) die entsprechenden Folgeprodukte in oft guten Ausbeuten erhalten werden. Sofern keine noch schneller mit Lithium-Metall reagierenden funktionellen Gruppen in den Reaktanden vorhanden sind, handelt es sich hierbei um eine Vorgehensweise, die sehr hohe Raum/Zeit-Ausbeuten liefert und zudem nur einen einzigen Kessel erfordert. In einigen Fällen können Raum/Zeit-Ausbeuten bis zu 0,3 kg Produkt/(L*h) erreicht werden.

Eine weitere bevorzugte Ausführungsform ist vor allem dann vorteilhaft, wenn die Eintopfvariante aus den oben genannten oder anderen Gründen nicht angewandt werden kann, und besteht in der primären quantitativen Herstellung der Lithiumverbindung und nachfolgender Umsetzung mit dem C-Elektrophil . In einer besonders bevorzugten Ausführungsform werden beide Stufen bei der gleichen oder einer nur geringfügig differierenden Temperatur durchgeführt, wobei hier zeitaufwendige und energiefressende Aufheiz- und Abkühlphasen vermieden werden.

Geeignete Lösungsmittel für die erfindungsgemäße Methode zur Knüpfung von C,C-Bindungen sind aliphatische und aromatische Ether und Kohlenwasserstoffe und Amine, die keinen Wasserstoff am Stickstoff tragen, bevorzugt Triethylamin, Diethylether, Tetrahydrofuran, Toluol, Toluol/THF-Mischungen, Anisol und Diisopropylether, besonders bevorzugt Toluol, THF oder Diisopropylether. Bevorzugt werden Lösungen in Konzentrationen im Bereich von 1 bis 60 Gew.-%, insbesondere 5 bis 40 Gew.-%, besonders bevorzugt 8 bis 30 Gew.-%.

Die erfindungsgemäßen Umwandlungen werden vorteilhaft bei Temperaturen zwischen -100 °C und +80 °C durchgeführt, bevorzugt bei -80 °C bis +20 °C, besonders bevorzugt zwischen -65 °C und -5 °C.

Das Lithium kann im vorliegenden Verfahren als Dispersion, Pulver, Späne, Sand, Granalien, Stücke, Barren oder in anderer Form eingesetzt werden, wobei die Größe der Lithiumpartikel nicht qualitätsrelevant ist, sondern lediglich die Reaktionszeiten beeinflusst. Daher sind kleinere Partikelgrößen bevorzugt, beispielsweise Granalien, Pulver oder Dispersionen. Die zugesetzte Lithiummenge beträgt je Mol umzusetzenden Halogens 1,95 bis 2,5 mol, bevorzugt 1,98 bis 2,15 Mol.

Die Aufarbeitung geschieht im allgemeinen wässrig, wobei entweder Wasser bzw. wässrige Mineralsäuren zudosiert werden oder die Reaktionsmischung auf Wasser bzw. wässrige Mineralsäuren dosiert wird. Zur Erzielung bester Ausbeuten wird hier jeweils der pH-Wert des zu isolierenden Produkts eingestellt, also für gewöhnlich ein leicht saurer, im Falle von N-Heterocyclen auch leicht alkalischer pH-Wert. Die alkylierten oder arylierten Produkte werden beispielsweise durch Extraktion und Eindampfen der organischen Phasen gewonnen, alternativ können auch aus der Hydrolysemischung die organischen Lösungsmittel abdestilliert und das dann ausfallende Produkt durch Filtration gewonnen werden.

Die Reinheiten der Produkte aus den erfindungsgemäßen Verfahren sind im allgemeinen hoch, für Spezialanwendungen (Pharmavorprodukte) kann allerdings noch ein weiterer Aufreinigungsschritt, beispielsweise durch Umkristallisation unter Zusatz geringer Mengen Aktivkohle, erforderlich werden. Die Ausbeuten an den Reaktionsprodukten betragen zwischen 70 und 99 %, typische Ausbeuten sind insbesondere 85 bis 95 %.

Die Rohstoffe für die erfindungsgemäße Synthese (Chloraromaten und Fluoraromaten) sind ganz allgemein sehr preisgünstig kommerziell erhältlich, so dass in Kombination mit den genannten verfahrenstechnischen Vorzügen und damit verbundenen hohen Raum/Zeit-Ausbeuten und sehr hohen Produktreinheiten ein äußerst ökonomischer und sehr generell anwendbarer Prozess zur erfindungsgemäßen Knüpfung von C,C-Bindungen gefunden wurde.

Das erfindungsgemäße Verfahren soll durch die nachfolgenden Beispiele erläutert werden, ohne die Erfindung darauf zu beschränken:

### Beispiele 1 bis 7

### Herstellung von p-Methylacetophenon aus Chlortoluol

Eine Mischung aus 126,5 g p-Chlortoluol (1 mol) und 45,1 g Acetonitril (frisch destilliert, 1,1 mol) wird zu einer Suspension von 13,8 g Lithiumgranalien (2,0 mol) in 350 ml THF bei -50°C zugetropft, wobei als Dosierzeit 2 Stunden gewählt wurde. Nach einem per GC bestimmten Umsatz (dunkle Farbe der Reaktionsmischung lässt Grad des Li-Verbrauchs nicht erkennen) von > 98 % (insgesamt 7,5 h) wird die Reaktionsmischung auf 200 g Wasser gegeben, der pH mit 37 % HCI auf 2,0 eingestellt und die Reaktionsmischung zwei Stunden lang am Rückfluss gekocht. Anschließend wird die organische Phase abgetrennt und die wässrige noch einmal mit 100 ml Petrolether extrahiert. Die vereinigten organischen Phasen werden destilliert. Man erhält 132,7 g 4-Methylacetophenon (0,99 mol, 99 %, Siedepunkt 88°C/8 Torr) als farblose Flüssigkeit, GC-Reinheit > 98 % a/a, die beim Stehen lassen bei RT allmählich erstarrt.

| | Lösungsmittel | Temperatur | Reaktionszeit | % Umsatz Metallierung |
|---|---|---|---|---|
| Beispiel 1 | THF | -50°C | 7,5 h | 98,5 |
| Beispiel 2 | THF | -60°C | 10 h | 98,3 |
| Beispiel 3 | THF/Toluol 1:1 | -40°C | 12 h | 95,5 |
| Beispiel 4 | THF (halbe Menge) | -50°C | 7,5 h | 96,8 |
| Beispiel 5 | Di-n-butyl-ether | -35°C | 14 h | 97,2 |
| Beispiel 6 | Diethylether | -25°C | 9 h | 98,3 |
| Beispiel 7 | Triethylamin | -65°C | 2 h | 98,2 |

### Beispiel 8

### Herstellung von p-Methylacetophenon aus Fluortoluol

Die Herstellung von p-Methylacetophenon aus p-Fluortoluol wurde wie in Beispiel 1 beschrieben durchgeführt, allerdings musste hier die Reaktionszeit bis zum Erreichen des erforderlichen Umsatzes verdoppelt werden. Die Hydrolyse wurde in einem Teflonkolben durchgeführt (HF). Man erhielt so 4-Methylacetophenon in einer Ausbeute von 88 %.

### Beispiel 9

### Herstellung von 2-(4-lsopropylphenyl)-propen aus 4-Chlorisopropylbenzol

154,5 g 4-Chlorisopropylbenzol (1 mol) werden in 1 h zu einer Suspension von 13,8 g Lithiumgranalien (2,0 mol) in 400 ml THF bei -45°C zugetropft. Nach einem per GC bestimmten Umsatz von > 97 % (insgesamt 9,5 h) wird die Reaktionsmischung in 30 Minuten zu einer Lösung von 60,9 g trockenem Aceton (1,05 mol) in 120 ml THF bei -45°C getropft und anschließend 1 h bei dieser Temperatur nachgerührt.
Die Reaktionsmischung wird in der in Beispiel 1 beschriebenen Weise hydrolysiert (150 ml Wasser, mit HCI auf pH 1,0 einstellen). Anschließend wird zur vollständigen Eliminierung von Wasser 6 h am Rückfluss gekocht. Anschließend werden bei Raumtemperatur die Phasen getrennt und die wässrige Phase noch einmal mit 100 ml Petrolether extrahiert. Die vereinigten organischen Phasen werden mit Natriumsulfat getrocknet und anschließend destillativ von verbliebenen Lösungsmitteln befreit. Es verbleiben 152 g 2-(4-lsopropylphenyl)-propen (0,95 mol, 95 %) als gelbliches Öl, GC-Reinheit 94,5 % a/a.

### Beispiel 10

### Herstellung von 2-(4-lsopropylphenyl)-propen aus 4-Fluorisopropylbenzol

Durchführung wie in Beispiel 3 beschrieben, Lithiierungszeit musste wiederum verdoppelt werden. Ausbeute 95 %, GC-Reinheit 93,1 % a/a.

### Beispiel 11

### Herstellung von 4-Octylbenzaldehyd aus 4-Chlorbenzaldehyd-Ethylenglykolacetal

184,5 g 4-Chlorbenzaldehyd-Ethylenglykolacetal (1 mol) werden in 2 h zu einer Suspension von 13,8 g Lithiumgranalien (2,0 mol) in 800 ml THF bei -50°C zugetropft. Nach einem per GC bestimmten Umsatz von > 97 % (insgesamt 8,5 h) werden in 60 Minuten 193 g n-Octylbromid (1,1 mol) bei -50°C zugetropft und anschließend 1 h bei dieser Temperatur nachgerührt.
Nach Hydrolyse (220 ml Wasser, mit HCI auf pH 2,0 einstellen) und 2 h Kochen am Rückfluss (zur Spaltung des Acetals) wird die organische Phase abgetrennt und die wässrige Phase noch einmal mit 100 ml Petrolether extrahiert. Die vereinigten organischen Phasen werden mit Natriumsulfat getrocknet und anschließend destillativ von verbliebenen Lösungsmitteln befreit. Es verbleiben 198,4 g 4-Octylbenzaldehyd (0,91 mol, 91 %) als leicht bräunliches Öl, GC-Reinheit 98 % a/a.

### Beispiel 12

### Herstellung von 4-(2-Hydroxyethyl)phenetol aus 4-Chlorphenetol

In 400 ml auf -60°C gekühltes THF werden 50 g Ethylenoxid (1,14 mol) einkondensiert. Anschließend werden unter Stickstoffatmosphäre zunächst 13,8 g Lithiumpulver (2,0 mol), dann in 10 Minuten 156,5 g 4-Chlorphenetol zugegeben. Nach Erreichen eines per GC bestimmten Umsatzes von > 96 % (insgesamt 5,5 h) wird die Reaktionsmischung in der in Beispiel 1 beschriebenen Weise hydrolysiert (150 ml Wasser, mit HCI auf pH 4,0 einstellen). Anschließend werden bei Raumtemperatur die Phasen getrennt und die wässrige Phase noch einmal mit 100 ml Dichlormethan extrahiert. Die vereinigten organischen Phasen werden destillativ von verbliebenen Lösungsmitteln und Wasser befreit. Es verbleiben 152,7 g 4-(2-Hydroxyethyl)phenetol (0,92 mol, 92 %) als hochviskose Flüssigkeit.

### Beispiel 13

### Herstellung von 4-Cyanobiphenyl

Unter Stickstoffatmosphäre werden 13,8 g Lithiumpulver (2,0 mol) in 225 ml THF vorgelegt. Eine Lösung von 188,5 g 4-Chlorbiphenyl und 119 g Phenylisocyanat in 225 ml THF wird im Laufe einer Stunde bei -65°C zugetropft. Nach Erreichen eines per HPLC bestimmten Umsatzes von > 95 % (insgesamt 8,5 h) wird die Reaktionsmischung in der in Beispiel 1 beschriebenen Weise hydrolysiert (150 ml Wasser, zur Abtrennung des Nebenproduktes Phenol belässt man den pH hier bei 12,4). Anschließend werden bei Raumtemperatur die Phasen getrennt und die wässrige Phase noch einmal mit 100 ml Dichlormethan extrahiert. Die vereinigten organischen Phasen werden noch einmal mit 3 % NaOH gewaschen und danach destillativ von verbliebenen Lösungsmitteln und Wasserresten befreit. Es verbleiben 166,5 g 4-Cyanobiphenyl (0,93 mol, 93 %) als farbloser Feststoff, der erforderlichenfalls aus Ethanol umkristallisiert werden kann.

### Beispiel 14

### Herstellung von 4-Hydroxymethyl-N,N-dimethylanilin aus 4-Chlor-N,N-dimethylanilin

In eine auf -50 °C gekühlte Vorlage mit 400 ml Toluol und 13,8 g Lithiumpulver (2,0 mol) dosiert man in 60 Minuten 155,5 g 4-Chlor-N,N-dimethylanilin. Nach Erreichen eines per GC bestimmten Umsatzes von > 98 % (insgesamt 6,5 h) wird in die Reaktionsmischung gasförmiger Formaldehyd eingeblasen, bis insgesamt 33 g aufgenommen wurden. Nach 30-minütigem Nachrühren wird in der beschriebenen Weise hydrolysiert (200 ml Wasser, mit HCI auf pH 7,0 einstellen). Anschließend werden bei Raumtemperatur die Phasen getrennt und die wässrige Phase noch zweimal mit jeweils 100 ml Dichlormethan extrahiert. Die vereinigten organischen Phasen werden destillativ von verbliebenen Lösungsmitteln und Wasser befreit. Es verbleiben 134,5 g 4-Hydroxymethyl-N,N-dimethylanilin (0,89 mol, 89 %) als viskose, bräunliche Flüssigkeit.

### Beispiel 15

### Herstellung von Acetophenonoxim

In eine auf -50°C gekühlte Vorlage mit 400 ml THF und 13,8 g Lithiumpulver (2,0 mol) dosiert man in 60 Minuten 112,5 g Chlorbenzol. Nach Erreichen eines per GC bestimmten Umsatzes von > 99 % (insgesamt 8,5 h) wird die grünliche Reaktionsmischung zu einer Suspension von Nitroethan-Enolat in THF dosiert (Herstellung: aus 1 mol Nitroethan, 1 mol n-BuLi in Hexan und 300 ml THF bei -50°C). Nach 30-minütigem Nachrühren wird in der beschriebenen Weise hydrolysiert (200 ml Wasser, mit HCI vorsichtig auf pH 6,0 einstellen). Anschließend werden bei Raumtemperatur die Phasen getrennt und die wässrige Phase noch einmal mit 100 ml Dichlormethan extrahiert. Die vereinigten organischen Phasen werden destillativ von verbliebenen Lösungsmitteln und Wasser befreit. Es verbleiben 101 g Acetophenonoxim (0,75 mol, 75 %) als gelbliches, hochviskoses Öl.

### Beispiel 16

### Herstellung von 1,2-Bis(4-methylthiophenyl)ethandion

In eine auf -70°C gekühlte Vorlage mit 400 ml THF und 13,8 g Lithiumpulver (2,0 mol) dosiert man in 60 Minuten 158,5 g 4-Chlorthioanisol. Nach Erreichen eines per GC bestimmten Umsatzes von > 96 % (insgesamt 7,5 h) wird in die rötliche Reaktionsmischung bei -75°C ein starker Strom von Kohlenmonoxid eingeblasen. Nach Ende der Aufnahme wird in der beschriebenen Weise hydrolysiert (200 ml Wasser, mit HCI vorsichtig auf pH 6,5 einstellen). Anschließend werden bei Raumtemperatur die Phasen getrennt und die wässrige Phase noch zweimal mit jeweils 100 ml Dichlormethan extrahiert. Die vereinigten organischen Phasen werden destillativ möglichst schonend von verbliebenen Lösungsmitteln und Wasser befreit. Es verbleiben nach Kristallisation aus Ethanol (96 %) 83 g 1,2-Bis(4-methylthiophenyl)ethandion (0,275 mol, 55 %) als brauner, unangenehm riechender Feststoff.

### Beispiel 17

### Herstellung von Bis(p-anisyl)carbinol

In eine auf -40°C gekühlte Vorlage mit 370 ml THF und 13,8 g Lithiumstücken (1 x cm x 1 cm x 0,5 cm, 2,0 mol) dosiert man in 30 Minuten 158,5 g 4-Chlorthioanisol (1,0 mol). Nach Erreichen eines per GC bestimmten Umsatzes von > 97 % (insgesamt 14,5 h) wird in die Reaktionsmischung bei -40°C eine Lösung von 58,8 g Ameisensäuremethylester (0,98 mol) im gleichen Volumen THF eingetropft. Nach einstündigem Nachrühren bei -40°C wird in der mehrfach beschriebenen Weise hydrolysiert (200 ml Wasser, mit HCI auf pH 6,5 einstellen). Anschließend werden bei Raumtemperatur die Phasen getrennt und die wässrige Phase noch dreimal mit jeweils 100 ml Dichlormethan extrahiert. Die vereinigten organischen Phasen werden destillativ von verbliebenen Lösungsmitteln und Wasser befreit. Es verbleiben 108,6 g Bis(p-Anisyl)carbinol (0,445 mol, 89 %) als gelbliches, viskoses Produkt.

### Beispiel 18

### Herstellung von 4-Methoxybiphenyl

In eine auf -50°C gekühlte Vorlage mit 370 ml THF und 13,8 g Lithiumstücken (1 x cm x 1 cm x 0,5 cm, 2,0 mol) dosiert man in 50 Minuten 142,5 g 4-Chloranisol (1,0 mol). Nach Erreichen eines per GC bestimmten Umsatzes von > 97 % (insgesamt 12,5 h) wird in die Reaktionsmischung bei -50°C zunächst eine Lösung von 0,05 g PdCl2(dppf) in 20 ml THF und anschließend in 30 Minuten eine Lösung von 157 g Brombenzol im gleichen Volumen THF eingetropft (stark exotherm). Nach einstündigem Nachrühren bei -50°C lässt man auf Raumtemperatur auftauen und hydrolysiert in der beschriebenen Weise (200 ml Wasser, mit HCI auf pH 5,5 einstellen). Anschließend werden bei Raumtemperatur die Phasen getrennt und die wässrige Phase noch einmal mit 150 ml Toluol extrahiert. Die vereinigten organischen Phasen werden destillativ von verbliebenen Lösungsmitteln und Wasser befreit. Es verbleiben 162 g 4-Methoxybiphenyl (0,88 mol, 88 %) als leicht gelblicher Feststoff, der destillativ weiter aufgereinigt werden kann.

| | Lösungsmittel | Temperatur | Reaktionszeit | % Ausbeute |
|---|---|---|---|---|
| Beispiel 8 | THF | -50°C | 15 h | 88 |
| Beispiel 9 | THF | -45°C | 9,5 h | 95 |
| Beispiel 10 | THF/Toluol 1:1 | -40°C | 24 h | 93,1 |
| Beispiel 11 | THF | -50°C | 8,5 h | 91 |
| Beispiel 12 | THF | -60°C | 5.5 h | 92 |
| Beispiel 13 | THF | -65°C | 8,5 h | 93 |
| Beispiel 14 | Toluol | - 50°C | 6,5 h | 89 |
| Beispiel 15 | THF | - 50°C | 8,5 h | 75 |
| Beispiel 16 | THF | - 70°C | 7,5 h | 96 |
| Beispiel 17 | THF | - 40°C | 14,5 h | 89 |
| Beispiel 18 | THF | - 50°C | 12,5 h | 88 |

### Beispiel 19

### Herstellung von 3-Acetylpyridin aus 3-Chlorpyridin

Eine Mischung aus 113,5 g 3-Chlorpyridin (1 mol) und 45,1 g Acetonitril (frisch destilliert, 1,1 mol) wird zu einer Suspension von 13,8 g Lithiumgranalien (2,0 mol) in 350 ml THF bei -75°C zugetropft, wobei als Dosierzeit 2 Stunden gewählt wurde. Nach einem per GC bestimmten Umsatz von > 95 % (insgesamt 17,5 h) wird die Reaktionsmischung in der schon mehrfach beschriebenen Weise aufgearbeitet. Nach Filtration der verbleibenden toluolischen Lösung über Primisil (high density-Zeolith) und Abkondensieren der Lösungsmittel verbleiben 110 g 3-Acetylpyridin (HPLC-Reinheit 93 %) als orangefarbene Flüssigkeit.

### Beispiel 20

### Herstellung von 3-Acetylfuran aus 3-Chlorfuran

Durch Umsetzung von 102,5 g 3-Chlorfuran (1 mol), 45,1 g Acetonitril (frisch destilliert, 1,1 mol) und 13,8 g Lithiumgranalien (2,0 mol) entsprechend der in Beispiel 19 angegebenen Vorschrift erhält man 98,5 g 3-Acetylfuran (HPLC-Reinheit 94 %) als hellbraune Flüssigkeit.

## Patentansprüche

1. Verfahren zur Herstellung von Verbindungen der Formel (II), 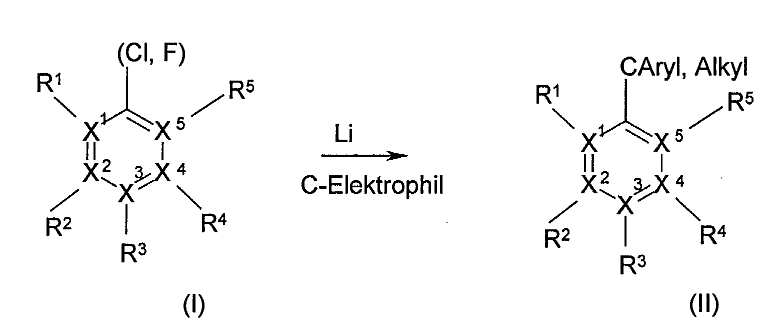 worin die Substituenten R¹ bis R⁵ unabhängig voneinander für H, CH₃, geradkettiges oder verzweigtes C₁-C₈-Alkyl, insbesondere C₁-C₄-Alkyl, CH(OC₁-C₅-Alkyl)₂, CH(C₁-C₅-Alkyl)(OC₁-C₅-Alkyl), CH₂(OC₁-C₅-Alkyl), CH(CH₃)(OC₁-C₅-Alkyl), C₁-C₈-Alkoxy, insbesondere C₁-C₄-Alkoxy, N(C₁-C₅-Alkyl)₂, Phenyl, substituiertes Phenyl, Aryl, Heteroaryl, S(C₁-C₅-Alkyl) oder für einen Rest C_{Aryl, Alkyl} stehen, und die Symbole X^{1 bis 5} für Kohlenstoff oder maximal zwei benachbarte X¹⁻⁵ für Stickstoff oder X¹R¹ und X²R² zusammen für O, NH, N(C₁-C₅-Alkyl), N(C=O-C₁-C₅-Alkyl), N(SiR₃)₂ oder S stehen,
oder wobei benachbarte Reste R¹ bis R⁵ folgende Struktureinheit bilden, 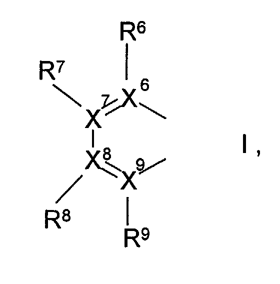 wobei X⁶ bis X⁹ und R⁶ bis R⁹ die gleiche Bedeutung haben wie X¹ bis X⁵ und R¹ bis
R⁵
und
der Rest C_{Aryl, Alkyl} für geradkettiges oder verzweigtes, substituiertes oder unsubstituiertes C₁-C₈-Alkyl, 1-Hydroxyalkyl mit 1 bis 8 C-Atomen, CN, 2-Hydroxyalkyl mit 2 bis 5 C-Atomen, 3-Hydroxyalkyl mit 3 bis 5 C-Atomen, 1-NHR-Alkyl mit 1 bis 5 C-Atomen, CH(OC₁-C₅-Alkyl)₂, C(C₁-C₅-Alkyl)(OC₁-C₅-Alkyl), CH₂(OC₁-C₅-Alkyl), CH(CH₃)(OC₁-C₅-Alkyl), C₁-C₅-Alkoxy, N(C₁-C₅-Alkyl)₂, Phenyl, substituiertes Phenyl, Aryl, Heteroaryl, CO₂H, CO₂Alkyl, (C=O)_{0.5}, substituierte 1-Vinylalkyle, CH₃-C(=O), R-C(=O) oder CHO steht, durch Umsetzung von Chlor- oder Fluoraromaten der Formel (I) mit Kohlenstoff-Elektrophilen (C-Elektrophilen) und Lithiummetall.

2. Verfahren nach Anspruch 1, **dadurch gekennzeichnet, dass** das Kohlenstoff-Elektrophil aus der folgenden Gruppe ausgewählt wird:
Aryl- oder Alkylcyanate (C_{Aryl,Alkyl} = CN)
Oxiran, substituierte Oxirane (C_{Aryl,Alkyl} = CH₂CH₂OH, substituierte CR₂CR₂OH) Azomethine (C_{Aryl,Alkyl} = CR¹₂-NR'H)
Nitroenolate (C_{Aryl,Alkyl} = Oxime)
Immoniumsalze (C_{Aryl,Alkyl} = Amine)
Halogenaromat, Aryltriflate, andere Arylsulfonate (C_{Aryl,Alkyl} = Aryl, Heteroaryl) Kohlendioxid (C_{Aryl,Alkyl} = COOH)
Kohlenmonoxid (C_{Aryl,Alkyl} = (-CO-)_{0,5})
Aldehyde, Ketone (C_{Aryl,Alkyl} = CHR¹-OH, CR¹₂-OH,
α,β-ungesättigte Aldehyde/Ketone (C_{Aryl,Alkyl} = CH(OH)-Vinyl, CR¹(OH)-Vinyl)
Ketene (C_{Aryl,Alkyl} = C(=O)CH₃ bei Keten, C(=O)-R bei substituierten Ketenen)
Alkali- und Erdalkalisalze von Carbonsäuren (C_{Aryl,Alkyl} = CHO bei Formiaten, COCH₃ bei Acetaten, R¹CO bei R¹COOMet)
Aliphatische Nitrile (C_{Aryl,Alkyl} = COCH₃ bei Acetonitril, R¹CO bei R¹CN)
Aromatische Nitrile (C_{Aryl,Alkyl} = COAr')
Amide (C_{Aryl,Alkyl} = CHO bei HCONR¹₂, C(=O)R¹ bei R¹CONR'₂)
Ester (C_{Aryl,Alkyl} = [C(OH)R¹]_{0,5}) oder
Alkylierungsmittel (C_{Aryl,Alkyl} = Alkyl).

3. Verfahren nach Anspruch 1 oder 2, **dadurch gekennzeichnet, dass** die Umsetzung bei einer Temperatur im Bereich von -100 bis +80°C durchgeführt wird.

4. Verfahren nach einem oder mehreren der vorhergehenden Ansprüche, **dadurch gekennzeichnet, dass** das Lithium in Form einer Dispersion, Pulver, Späne, Sand, Granalien, Stücke oder in Form von Barren eingesetzt wird.

5. Verfahren nach einem oder mehreren der vorhergehenden Ansprüche, **dadurch gekennzeichnet, dass** als Lösungsmittel aliphatische und aromatische Ether, Kohlenwasserstoffe und Amine, die keinen Wasserstoff am Stickstoff tragen, bevorzugt Triethylamin, Diethylether, Tetrahydrofuran, Toluol, Toluol/THF-Mischungen, Anisol und Diisopropylether, besonders bevorzugt Toluol, THF oder Diisopropylether, eingesetzt werden.

6. Verfahren nach einem oder mehreren der vorhergehenden Ansprüche, **dadurch gekennzeichnet, dass** das Verfahren als Eintopfverfahren durchgeführt wird.

7. Verfahren nach einem oder mehreren der vorhergehenden Ansprüche, **dadurch gekennzeichnet, dass** zunächst die lithiumorganische Verbindung generiert wird und diese bei der gleichen oder einer geringfügig differierenden Temperatur mit dem C-Elektrophil umgesetzt wird.
